# EUROPÄISCHE PATENTSCHRIFT

(11) **EP 1 049 490 B1**
(45) Veröffentlichungstag und Bekanntmachung des Hinweises auf die Patenterteilung: **31.07.2002**
(21) Anmeldenummer: 99907249.9
(22) Anmeldetag: 15.01.1999
(51) Int. Cl.: A61K 39/395, C07K 14/705, C07K 14/52, C07K 16/28

(54) **INHIBIERUNG VON CD95-UNABHÄNGIGER APOPTOSE BEI AIDS**
METHOD FOR INHIBITING CD95-INDEPENDENT APOPTOSIS IN AIDS
INHIBITION D'APOPTOSE CD95-INDEPENDANTE DANS LE SIDA

(30) Priorität: 15.01.1998 DE 19801265
(43) Veröffentlichungstag der Anmeldung: 08.11.2000
(73) Patentinhaber: Deutsches Krebsforschungszentrum Stiftung des öffentlichen Rechts, 69120 Heidelberg (DE)
(72) Erfinder: KRAMMER, Peter, H., D-69120 Heidelberg (DE); BERNDT, Christina, D-69115 Heidelberg (DE)
(74) Vertreter: Schüssler, Andrea, Dr.
(86) Internationale Anmeldenummer: DE9900109
(87) Internationale Veröffentlichungsnummer: WO9936091

(56) Entgegenhaltungen:
- File Medline, Zusammenfassung n. 95171468, 1995 XP002107782 & M RADRIZZANI ET AL.: "IL-12 inhibits apoptosis induced in a human Th1 clone by gp120/CD4 cross-linking and CD3/TCR activation or by IL-2 deprivation" CELL. IMMUNOL., Bd. 161, Nr. 1, März 1995, Seiten 14-21,
- J CORBEIL & D D RICHMAN: "Productive infection and subsequent interaction of CD4-gp120 at the cellular membrane is required for HIV-induced apoptosis of CD4+ T cells" JOURNAL OF GENERAL VIROLOGY., Bd. 76, 1995, Seiten 681-690, XP002107780 READING GB
- C BERNDT ET AL.: "CXCR4 and CD4 mediate a rapid CD95-independent cell death in CD4+ T cells" PROCEEDINGS OF THE NATIONAL ACADEMY OF SCIENCES OF USA., Bd. 95, Nr. 21, 13. Oktober 1998, Seiten 12556-12561, XP002107781 WASHINGTON US

## Beschreibung

Die vorliegende Erfindung betrifft ein Verfahren zur Inhibierung von CD95-unabhängiger Apoptose, insbesondere bei AIDS. Ferner betrifft die Erfindung ein System zur Identifizierung von Substanzen, die zur Inhibierung von CD95-unabhängiger Apoptose verwendet werden können.

AIDS kennzeichnet sich durch eine Depletion von CD4⁺T-Zellen und eine Dysfunktion des Immunsystems. Bisherige Daten weisen darauf hin, daß die Depletion eintritt, weil in Gegenwart des Glykoproteins gp120 von HIV der CD95-Rezeptor durch den in erhöhter Menge vorliegenden CD95-Liganden aktiviert wird, wodurch eine Apoptose, d.h. programmierter Zelltod, in CD4⁺T-Zellen induziert wird. Diese Apoptose wird mit CD95-vermittelter Apoptose bezeichnet.

Versuche wurden unternommen, die Depletion von CD4⁺T-Zellen bei AIDS zu verhindern. Hierzu wurden Verfahren mit Substanzen durchgeführt, welche die Bindung des CD95-Liganden an den CD95-Rezeptor blockieren. Diese Verfahren führten allerdings nicht zu einer vollständigen Verhinderung der Depletion von CD4⁺T-Zellen.

Der vorliegenden Erfindung liegt somit die Aufgabe zugrunde ein Verfahren bereitzustellen, mit dem die Depletion von CD4⁺T-Zellen bei AIDS untersucht und ggfs. verhindert werden kann.

Erfindungsgemäß wird dies durch die Gegenstände in den Patentansprüchen erreicht.

Gegenstand der vorliegenden Erfindung ist somit ein Verfahren zur Inhibierung in-vitro von CD95-unabhängiger Apoptose, wobei das Verfahren die Verfahrensschritte umfaßt:
(a) Blockierung der Bindung von HIV-1 gp120 an die Rezeptoren CD4 und/oder CXCR4 bzw. Blockierung eines um diese Bindung konkurrierenden Faktors, und/oder
(b) Inhibierung des durch die Bindung von (a) induzierten Signalwegs.

Die vorliegende Erfindung beruht auf der Erkenntnis des Anmelders, daß in Zellen nicht nur eine CD95-vermittelte Apoptose, sondern auch eine CD95-unabhängige Apoptose auftreten kann. Er hat gefunden, daß die CD95-unabhängige Apoptose z.B. dadurch induziert wird, daß das Glykoprotein gp120 von HIV-1 an die Rezeptoren CD4 und/oder CXCR4 bindet. Diese Rezeptoren liegen in verschiedenen Zellen vor. Ferner hat der Anmelder erkannt, daß die Rezeptoren CD4 und CXCR4 auch durch andere Faktoren aktiviert werden können, wodurch Apoptose induziert wird. Diese Faktoren sind insbesondere solche, die mit HIV-1 gp120 um die Bindung an die Rezeptoren CD4 und CXCR4 konkurrieren. Des weiteren hat der Anmelder gefunden, daß die CD95-unabhängige Apoptose sich in verschiedenen Eigenschaften von der CD95-vermittelten Apoptose unterscheidet. Einige dieser Eigenschaften sind in der nachstehenden Tabelle 1 aufgeführt.

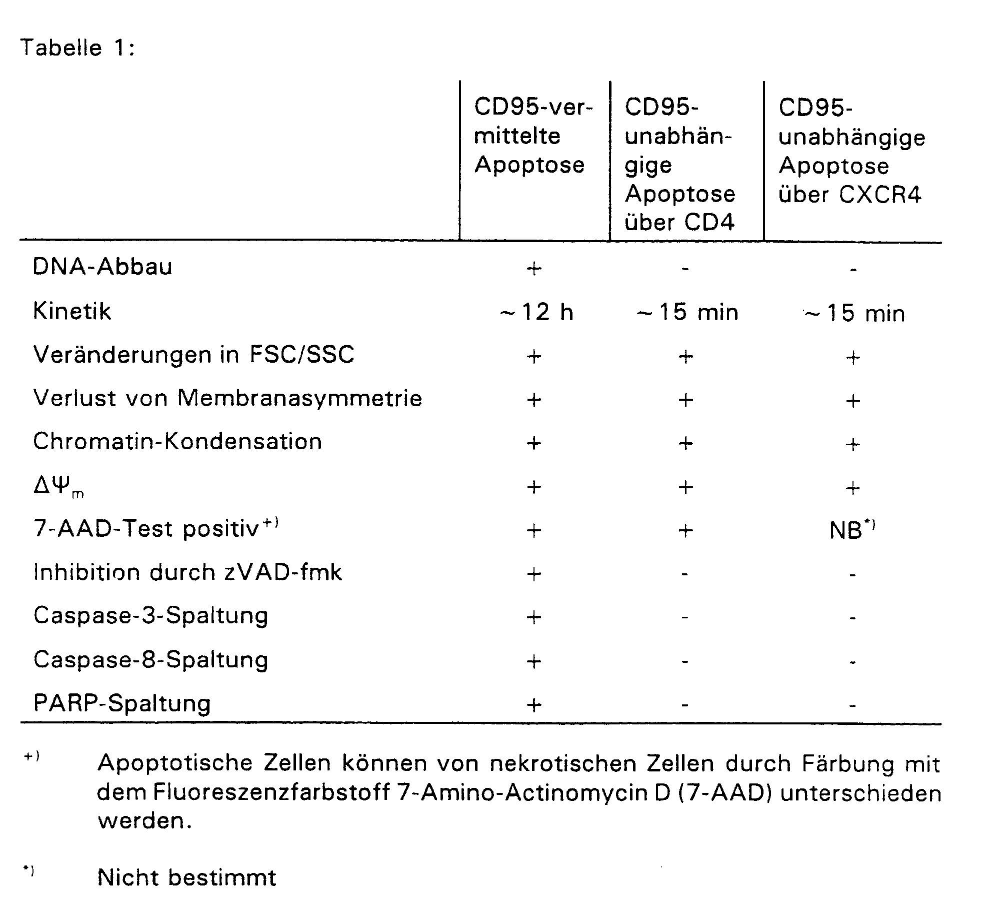

Erfindungsgemäß werden die vorstehenden Erkenntnisse für ein Verfahren genutzt, mit dem CD95-unabhängige Apoptose inhibiert werden kann. Ein solches Verfahren umfaßt die Verfahrensschritte:
(a) Blockierung der Bindung von HIV-1 gp120 an die Rezeptoren CD4 und/oder CXCR4 bzw. Blockierung eines um diese Bindung konkurrierenden Faktors, und/oder
(b) Inhibierung des durch die Bindung von (a) induzierten Signalwegs.

Der Ausdruck "Rezeptoren CD4 und/oder CXCR4" weist darauf hin, daß diese Rezeptoren in Zellen jeglicher Art und Herkunft vorliegen können. Insbesondere können es Zellen sein, in denen CD4 und/oder CXCR4 von Natur aus vorliegen. Beispiele solcher Zellen sind CD4⁺/CXCR4⁺-T-Zellen, wie HPB-ALL-Zellen. Auch können es Zellen sein, in denen CD4 und/oder CXCR4 von Natur aus nicht vorliegen, jedoch künstlich eingebracht worden sind. Beispiele solcher Zellen sind lymphoblastoide B-Zellen, wie BJAB-Zellen. Des weiteren umfaßt der Ausdruck "Rezeptoren CD4 und/oder CXCR4" auch diejenigen Teile dieser Rezeptoren, an die HIV-1 gp120 oder ein damit konkurrierender Faktor binden kann.

Der Ausdruck "ein um die Bindung konkurrierender Faktor" umfaßt jeglichen Faktor, der mit HIV-1 gp120 um die Bindung an die Rezeptoren CD4 und/oder CXCR4 konkurrieren kann. Insbesondere kann der Faktor ein anti-CD4-Antikörper, wie HP2/6 (Carrera, Universität Madrid) oder IOT4A (Immunotech) sein. Auch kann der Faktor ein anti-CXCR4-Antikörper, wie 12G5 (R + D Systems), sein. Ferner kann der Faktor ein anderes HIV-Protein, z.B. das Glykoprotein gp160, sein. Ein vorstehendes HIV-Protein kann von jeglichem HIV-Stamm, insbesondere von HIV-1 oder HIV-2, stammen. Des weiteren kann der Faktor ein Protein sein, das von einem HIV-Protein abgeleitet ist. Ein solches Protein kann z.B. die Region umfassen, die in HIV-Proteinen für die Bindung an die Rezeptoren CD4 und CXCR4 verantwortlich ist.

Der Ausdruck "Blockierung der Bindung" umfaßt jegliche Art und jegliches Mittel, mit denen die Bindung von HIV-1 gp120 an die Rezeptoren CD4 und/oder CXCR4 bzw. eines um diese Bindung konkurrierenden Faktors blockiert werden kann. Insbesondere kann das Mittel der natürliche Ligand von CD4, wie ein MHC-Molekül, sein. Auch kann das Mittel der natürliche Ligand von CXCR4, wie SDF-1α, sein. Günstig kann es sein, die natürlichen Liganden von CD4 und CXCR4 gemeinsam zu verwenden.

Der Ausdruck "Inhibierung des Signalwegs" umfaßt jegliche Art und jegliches Mittel, mit denen der Signalweg inhibiert werden kann, der durch die Bindung von HIV-1 gp120 an die Rezeptoren CD4 und/oder CXCR4 bzw. eines um diese Bindung konkurrierenden Faktors induziert wird. Insbesondere kann das Mittel ein solches sein, mit dem der Verlust von Membran-Asymmetrie verhindert werden kann. Auch kann das Mittel ein solches sein, mit dem die Kondensation von Chromatin verhindert werden kann.

Erfindungsgemäß wird auch ein Kit bereitgestellt, mit dem CD95-unabhängige Apoptose untersucht werden kann. Ferner können mit diesem System Substanzen identifiziert werden, die sich zur Inhibierung von CD95-unabhängiger Apoptose eignen. Ein solches System umfaßt Zellen, die CD4+ und/oder CXCR4+ sind, und HIV-1 gp120 sowie einen Faktor, der mit HIV-1 gp120 um die Bindung an CD4+ und/oder CXCR4+ konkurriert, sowie ggfs. eine Substanz, deren Inhibitionswirkung auf die Bindung von HIV-1 gp120 an die Rezeptoren CD4 und/oder CXCR4 oder eines um diese Bindung konkurrierenden Faktors bzw. auf den durch diese Bindung induzierten Signalweg bestimmt werden soll. Günstig kann es sein, wenn das System auch eine Substanz enthält, deren Inhibitionswirkung bekannt ist. Hinsichtlich solcher Substanzen wie auch der anderen Komponenten des Systems gelten vorstehende Ausführungen entsprechend.

Mit der vorliegenden Erfindung ist es möglich, CD95-unabhängige Apoptose zu untersuchen und zu beeinflussen. Insbesondere ist es möglich, CD95-unabhängige Apoptose zu inhibieren. Andererseits weist die Erfindung auch auf Möglichkeiten hin, CD95-unabhängige Apoptose zu induzieren. Solche Möglichkeiten ergeben sich u.a. durch die Verwendung von Faktoren, die mit der Bindung von HIV-1 gp120 an die Rezeptoren CD4 und/oder CXCR4 konkurrieren. Solche Faktoren sind insbesondere Antikörper, die gegen CD4 bzw. CXCR4 gerichtet sind. Des weiteren ermöglicht die vorliegende Erfindung Substanzen zu identifizieren, die CD95-unabhängige Apoptose beeinflussen, insbesondere inhibieren können.

Somit stellt die vorliegende Erfindung die Möglichkeit dar, CD95-unabhängige Apoptose zu inhibieren bzw. zu induzieren. Damit liefert die vorliegende Erfindung ein Mittel, die Depletion von CD4⁺-T-Zellen bei AIDS zu verhindern. Ferner stellt die vorliegende Erfindung ein Mittel dar, CD95-unabhängige Apoptose gezielt zu induzieren, was insbesondere bei der Bekämpfung von Tumorzellen von Bedeutung ist.

Die Erfindung wird durch das nachstehende Beispiel erläutert.

### Beispiel:

### Induktion von CD95-unabhängiger Apoptose und ihre Inhibierung

### I. Induktion von CD95-unabhängiger Apoptose

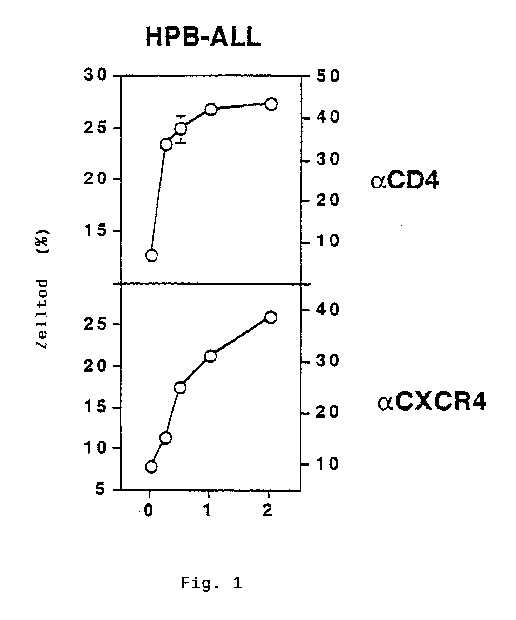

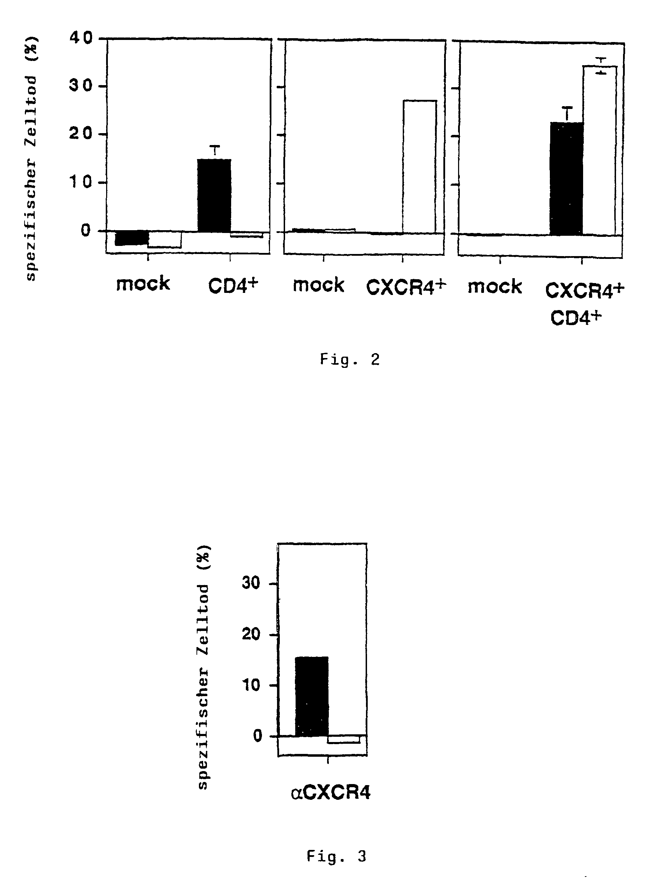

(a) Es werden CD4⁺/CXCR4⁺-Zellen (HPB-ALL-Zellen) verwendet. HPB-ALL-Zellen sind CD95⁻, d.h. in ihnen tritt keine CD95-vermittelte Apoptose auf.
   1 x 10⁵-HPB-ALL-Zellen werden 15 Minuten mit 5 µg/ml Maus-anti-CD4-Antikörper(HP2/6) bzw. Maus-anti-CXCR4-Antikörper (12G5) bei 37 °C inkubiert. Dann werden die gebundenen Antikörper mit Schaf-anti-Maus-Antikörpern (100 µg/ml, Boehringer) umgesetzt. Es wird eine Apoptose-Bestimmung durchgeführt, in der eine FSC/SSC-Analyse in einem FACScan-Cytometer zu verschiedenen Zeitpunkten gefahren wird (vgl. Fig. 1).
   Es zeigt sich, daß die Antikörper anti-CD4 bzw. anti-CXCR4 jeweils eine CD95-unabhängige Apoptose induzieren können.
(b) Es werden CD4⁻/CXCR4⁻-Zellen (BJAB-Zellen) verwendet. In diese Zellen werden die Rezeptoren CD4 und CXCR4 eingebracht, indem für die Rezeptoren kodierende DNA-Konstrukte in die Zellen transfiziert und dann exprimiert werden.
   BJAB-Zellen werden mit 2 µg des Hygromycin-Resistenz-Vektors pKEX2XL und 10 µg des Vektors pCDM8-CD4 (Kolanus, Universität München) transfiziert. Zellklone werden in Gegenwart von Hygromycin B (450 U/ml) selektiert. Ausgewählte Zellklone bzw. BJAB-Zellen werden ferner mit 10 µg des Vektors pcDNA3-tag-CXCR4 (Moulard, Universität Marseille) transfiziert. Zellklone werden in Gegenwart von G 418 (4 mg/ml) selektiert. Ausgewählte Zellklone von beiden Transfektionsrunden werden auf die Expression der entsprechenden Rezeptoren getestet. Zellklone, die CD4, CXCR4 bzw. CD4 und CXCR4 exprimieren, werden einer Apoptose-Induktion, wie unter (a) beschrieben, unterzogen (vgl. Fig. 2).
   Es zeigt sich, daß CD95-unabhängige Apoptose in Zellen induziert werden kann, in die CD4 und/oder CXCR4 künstlich eingebracht worden sind.

### II. Inhibierung von CD95-unabhängiger Apoptose

HPB-ALL-Zellen werden mit 250 nM SDF-1α 30 Minuten bei 37 °C inkubiert. Die Zellen werden dann einer Apoptose-Induktion mit dem Mausanti-CXCR4-Antikörper (12G5), wie vorstehend unter I. (a) beschrieben, unterzogen.

Es zeigt sich, daß mit dem natürlichen Liganden von CXCR4, SDF-1α, die Induktion von CD95-unabhängiger Apoptose verhindert werden kann.

## Patentansprüche

1. Verfahren zur in-vitro Inhibierung von CD95-unabhängiger Apoptose, umfassend die Verfahrensschritte:
(a) Blockierung der Bindung von HIV-1 gp120 an die Rezeptoren CD4 und/oder CXCR4 bzw. eines um diese Bindung konkurrierenden Faktors, und/oder
(b) Inhibierung des durch die Bindung von (a) induzierten Signalwegs.

2. Verfahren nach Anspruch 1, wobei der konkurrierende Faktor ein anti-CD4-Antikörper ist.

3. Verfahren nach Anspruch 1, wobei der konkurrierende Faktor ein anti-CXCR4-Antikörper ist.

4. Verfahren nach einem der Ansprüche 1 - 3, wobei die Blockierung von (a) durch den natürlichen Liganden von CD4 und/oder CXCR4 erfolgt.

5. Verfahren nach Anspruch 4, wobei der natürliche Ligand von CD4 ein MHC-Molekül ist.

6. Verfahren nach Anspruch 4, wobei der natürliche Ligand von CXCR4 SDF-1α ist.

7. Kit zur Identifizierung von Substanzen, die sich zur Inhibierung von CD95-unabhängiger Apoptose eignen, umfassend Zellen, die CD4⁺ und/oder CXCR4⁺ sind, und HIV-1 gp120 sowie einen Faktor, der mit HIV-1 gp120 um die Bindung an CD4⁺ und/oder CXCR4⁺ konkurriert.

8. Kit nach Anspruch 7, wobei das System eine Substanz enthält, deren Inhibitionswirkung auf die Bindung von HIV-1 gp120 an die Rezeptoren CD4 und/oder CXCR4 oder eines um diese Bindung konkurrierenden Faktors bzw. auf den durch diese Bindung induzierten Signalweg bestimmt werden soll.

9. Kit nach Anspruch 7 oder 8, wobei der konkurrierende Faktor ein anti-CD4-Antikörper ist.

10. Kit nach Anspruch 7 oder 8, wobei der konkurrierende Faktor ein anti-CXCR4-Antikörper ist.

## Claims

1. A method of in-vitro inhibiting CD95-independent apoptosis, comprising the steps of:
(a) blocking the bonding of HIV-1 gp120 to receptors CD4 and/or CXCR4 and of a factor competing for this bonding, respectively, and/or
(b) inhibiting the signal path induced by the bonding of (a).

2. The method according to claim 1, wherein the competing factor is an anti-CD4 antibody.

3. The method according to claim 1, wherein the competing factor is an anti-CXCR4 antibody.

4. The method according to any or claims 1 to 3, wherein (a) is blocked by the natural ligand of CD4 and/or CXCR4.

5. The method according to claim 4, wherein the natural ligand of CD4 is an MHC molecule.

6. The method according to claim 4, wherein the natural ligand of CXCR4 is SDF-1α.

7. Kit for identifying substances suitable to inhibit CD95-independent apoptosis, comprising cells, which are CD4⁺ and/or CXCR4⁺, and HIV-1 gp120 as well as a factor competing with HIV-1 gp120 for the bonding to CD4⁺ and/or CXCR4⁺.

8. Kit according to claim 7, wherein the system contains a substance whose inhibitory effect shall be determined on the bonding of HIV-1 gp120 to receptors CD4 and/or CXCR4 or a factor competing for this bonding and on the signal path induced by this bonding, respectively.

9. Kit according to claim 7 or 8, wherein the competing factor is an anti-CD4 antibody.

10. Kit according to claim 7 or 8, wherein the competing factor is an anti-CXCR4 antibody.

## Revendications

1. Procédé d'inhibition *in vitro* d'apoptose indépendante de CD95, comprenant les étapes suivantes :
(a) blocage de la liaison de la glycoprotéine gp120 de HIV-1 aux récepteurs CD4 et/ou CXCR4 ou d'un facteur en concurrence pour cette liaison, et/ou
(b) inhibition du parcours du signal induit par la liaison de (a).

2. Procédé suivant la revendication 1, dans lequel le facteur en concurrence est un anticorps anti-CD4.

3. Procédé suivant la revendication 1, dans lequel le facteur en concurrence est un anticorps anti-CXCR4.

4. Procédé suivant l'une des revendications 1 à 3, dans lequel le blocage de
(a) est accompli par les ligands naturels de CD4 et/ou de CXCR4.

5. Procédé suivant la revendication 4, dans lequel le ligand naturel de CD4 est une molécule de CMH.

6. Procédé suivant la revendication 4, dans lequel le ligand naturel de CXCR4 est le SDF-1α.

7. Kit d'identification de substances qui conviennent pour l'inhibition d'apoptose indépendante de CD95, comprenant des cellules qui sont CD4⁺ et/ou CXCR4⁺ et la glycoprotéine gp120 de HIV-1 ainsi qu'un facteur en concurrence avec la glycoprotéine gp120 de HIV-1 pour la liaison à CD4⁺ et/ou CXCR4⁺.

8. Kit suivant la revendication 7, dans lequel le système contient une substance dont l'action inhibitrice sur la liaison de la glycoprotéine gp120 de HIV-1 aux récepteurs CD4 et/ou CXCR4 ou d'un facteur en concurrence pour cette liaison ou sur le parcours du signal induit par cette liaison, doit être déterminée.

9. Kit suivant la revendication 7 ou 8, dans lequel le facteur en concurrence est un anticorps anti-CD4.

10. Kit suivant la revendication 7 ou 8, dans lequel le facteur en concurrence est un anticorps anti-CXCR4.
